# EUROPEAN PATENT SPECIFICATION

(11) **EP 2 185 233 B1**
(45) Date of publication and mention of the grant of the patent: **01.04.2020**
(21) Application number: 08737275.1
(22) Date of filing: 14.04.2008
(51) Int. Cl.: A61M 25/00, A61M 25/10, A61M 29/02, A61L 29/08, A61L 29/16

(54) **ANTI-RESTENOSIS DRUG COVERED AND ELUTING BALLOONS FOR VALVULOPLASTY OF AORTIC VALVE STENOSIS FOR THE PREVENTION OF RESTENOSIS**
MIT EINEM RESTENOSE VERHINDERNDEN ARZNEIMITTEL BESCHICHTETE UND DIESES FREISETZENDE BALLONS FÜR VALVULOPLASTIE BEI AORTENKLAPPENSTENOSE ZUR PRÄVENTION VON RESTENOSE
BALLONNETS À ÉLUTION RECOUVERTS D'UN MÉDICAMENT ANTI-RESTÉNOSE POUR VALVULOPLASTIE DE LA STÉNOSE DE LA VALVULE AORTIQUE, DANS LA PRÉVENTION DE LA RESTÉNOSE

(30) Priority: 13.04.2007 GR 20070100224
(43) Date of publication of application: 19.05.2010
(73) Proprietor: Eurocor Tech GmbH, 53117 Bonn (DE)
(72) Inventor: Spargias, Konstantinos, 171 21 N. Smirni Attikis (GR)
(74) Representative: Schneiders & Behrendt PartmbB Rechtsanwälte - Patentanwälte
(86) International application number: PCT/GR2008/000024
(87) International publication number: WO 2008/125890

(56) References cited:
- EP-A- 0 713 712
- WO-A-99/32184
- WO-A-2005/007219
- US-A- 5 120 322
- US-B1- 6 296 660
- US-B1- 6 364 856

## Description

### Background. Where aortic valvuloplasty stands today

Although aortic valve surgery is the gold standard, life saving treatment for symptomatic aortic stenosis, some patients do not undergo surgery. According to the 2003 Euro Heart Survey (lung B, et al, Eur Heart J 2003;24:1231-43) one third of community patients above the age of 75 with symptomatic severe aortic stenosis do not undergo surgery. The most apparent reason for this is the high mortality of the aortic valve replacement (AVR) in this population. The Medicare data in 2003 found a 8.8% in-hospital mortality of AVR in 145 000 patients aged over 65. There are other data showing average mortality of AVR up to 20% in octogenarians.

The incidence of known and severe aortic stenosis in US population in 2006 was 318,000 cases, of which only 74,000 had undergone AVR (based on Nkomo et al, Lancet 2006). According to other US estimates for 2006, of the 161,107 cases of ECHO diagnosed severe aortic stenosis over a 10-year period only 61,658 undergone AVR (based on Loma Linda data). Given the bad prognosis of severe symptomatic aortic stenosis, there has been an unmet need for another supplementary/alternative therapy.

Recently there has been considerable change in the technical approach to balloon aortic valvuloplasty (BAV) owing to the progress of percutaneous aortic valve implantation retrograde from the femoral artery and advances in the interventional hardware. A decrease in the complication rate of BAV over the past 15 years has been reported and has important implications regarding the morbidity associated with percutaneous aortic valve replacement techniques. In a recent series of 104 BAV procedures there were no procedural deaths and the incidence of total vascular complications was 9%. In-hospital, 1-, 2- and 3-year mortality rates were 6%, 44%, 62% and 71%, respectively. In the 1980's NHLBI Balloon Valvuloplasty Registry the procedural mortality was 3% and the in-hospital mortality 11%.

BAV has been well demonstrated to moderately increase aortic valve area by an average of 0.3-0.4 cm² and reduce mean valvular gradient by roughly 50%. This is achieved despite the use of balloon diameters of 18-24mm, not far smaller from the diameter of the aortic valve ring. Transient valve leaflet and annular stretch undoubtedly contribute to early recoil. The large majority of patients experience immediate symptomatic improvement.

However, the restenosis rates of 42-83% at 5-9 months and consistently >80% at 1 year remain the Achilles heel of the method and is the reason of the dismal long-term event-free and actuarial survival after balloon aortic valvuloplasty that resembles the natural history of untreated aortic stenosis.

It is now recognized that calcific aortic stenosis is a complex cellular process with features of atherosclerosis and biomineralization similar to osteogenesis, which should have specific pathways for targeted inhibition. Similarly, regulated processes may play a role in restenosis following BAV. Although the mechanisms of restenosis are poorly understood, scar formation and heterotopic ossification are believed to play a central role. If specific targeted inhibition of these processes is completely or even partially successful the long-term results of the BAV procedure will improve and it may become an acceptable treatment of aortic valve stenosis in the elderly.

An increasing number of patients are living into the 80s and beyond. Although many of these patients who may have symptomatic stenosis are acceptable candidates for surgical treatment, many are not. Given the uniform desire on behalf of the elderly for improvement in their quality of life, any advance in the BAV procedure that will limit restenosis and improve its long-term results would be welcome in this population.

The first effort to prevent restenosis following BAV was with radiation. The results of the RADAR pilot trial were published in Cathet Cardiovasc Intervent 2006;68:183-92. This was a series of 20 patients over 80 years of age with an estimated operative mortality risk above 15%. They underwent prophylactic external beam radiation therapy (EBRT) starting the day following the BAV procedure and for 3 days. A total dose of 1200 cGy and 1500-1800 cGy was administered in the low and high dose groups of patients. Restenosis was defined as over 50% late loss of acute gain in aortic valve area. By 1 year the restenosis rate in the low dose group was 30% and in the high dose group 11%, results impressively better than historical controls.

WO2005/007219 A discloses a balloon for use in valvuloplasty of aortic stenosis according to the preamble of claim 1.

### Description of the invention

The restenosis in coronary arteries and aortic valve following dilatation of a balloon share certain pathophysiological aspects. My hypothesis is that local antiproliferative drug delivery at the stenotic aortic valve with a balloon is a simple and effective way of preventing restenosis. If the restenosis problem is solved or even attenuated the acceptance/usage of this therapy would be completely revived.

I describe the development of drug-coated balloon for BAV, which elutes the loaded drug to the aortic valve tissues upon contact. There are numerous possibilities to drug coatings for this balloon, from drugs inhibiting scar formation to drugs inhibiting heterotopic ossification.

Since the mechanism of valve restenosis following BAV share certain characteristics with the coronary in-stent restenosis, drugs used for prevention of the latter can be tested for efficacy in the former. Paclitaxel for example is known to inhibit fibroblast migration in vitro and in vivo, and can be the first one to be used for coating a BAV balloon.

Recently, a pilot trial of external beam radiation therapy (EBRT) following BAV in 20 patients demonstrated a sustained significant improvement in the aortic valve area and mean gradient up to 1 year after the procedure (in reality there were only 2 outliers). However, this preventive strategy, even if it is conclusively proved, has certain inherent disadvantages such as the need for liaison with other medical specialties and medical facilities, precision of targeting, and prolonged hospital stay. The effort of restenosis prevention with radiation is reminiscent of the usage of brachytherapy for in-stent restenosis in coronary arteries, before the advent of drug-eluting stents.

In the event that antiproliferative drugs, such as paclitaxel, do not prove to be sufficiently effective, the component of valve restenosis due to heterotopic ossification could be dealt with incorporation in the coating of inhibitors of mineralization/calcification such as MGP, fetuin, osteopontinad and others or oral administration of inhibitors such as phosphate binding drugs (used by all chronic renal failure patients) and NSAA (i.e. the selective cyclooxygenase-2 (COX-2) inhibitor Rofecoxib).

An effective drug-coated balloon for BAV would prevent restenosis with local drug delivery at the aortic valve leaflets at the time of balloon inflation. This method is obviously superior to EBRT, which requires prolonged hospital stay and considerable material and human resources.

If such a balloon proves to prevent restenosis and offers a long-term symptomatic improvement, it will undoubtedly become the treatment of choice in the elderly. The procedure will be much simpler, safer and cheaper compared to the implantation of a percutaneous prosthetic valve.

Even partial success with reduction and delay but not elimination of restenosis will be welcome, since repeat BAV can be done in those in need. It is known that repeat BAV is feasible, safe and offers further relief, but cannot be seen as the solution when the restenosis rate is close to 100% after 1 year. However, if the use of the drug-coated balloon attenuates and cuts down the restenosis to acceptable rates, BAV with drug-coated balloon will become readily acceptable.

DIOR (Eurocor, Germany) is a balloon catheter coated with paclitaxel (3 mcg/mm² of balloon surface area) for use in the coronary arteries. It releases 35% of the drug with every 20-second contact with the vessel wall (i.e. 2 such dilatations release almost 70% of the loaded drug). It has been shown to significantly reduce late lumen loss and coronary in-stent restenosis compared with an uncoated balloon.

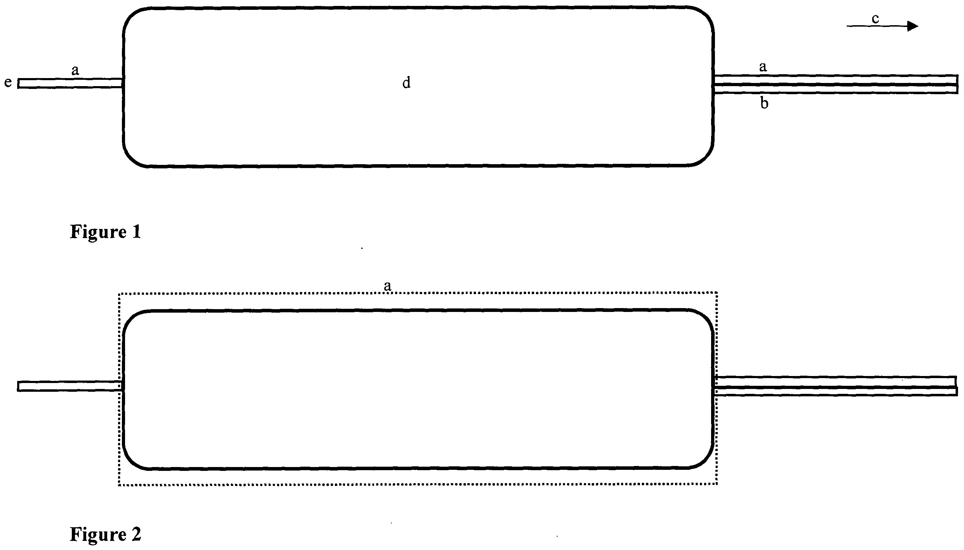

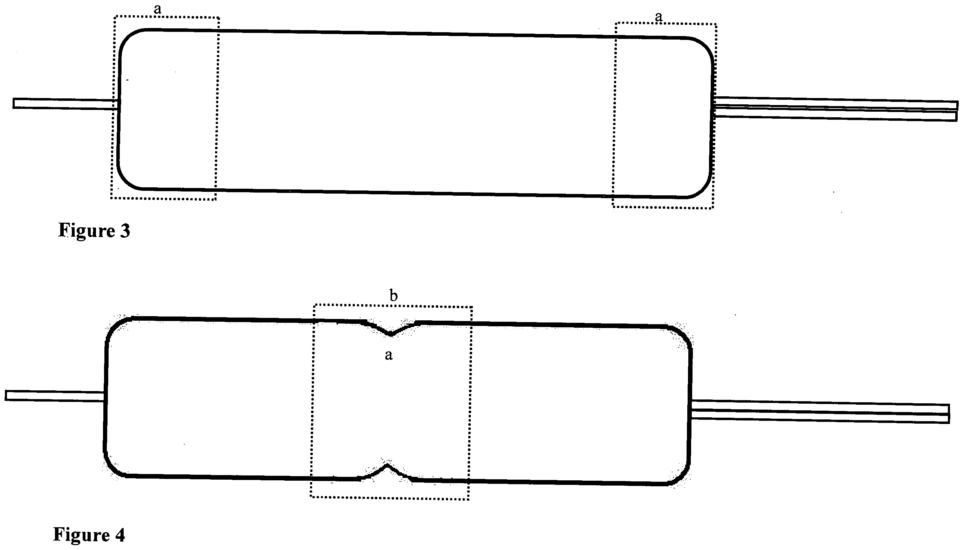

The aortic valvuloplasty balloon first introduced in the late 70s and today there are plenty plain valvuloplasty balloons manufacturers for use in heart valves. The present invention provides a balloon for use in valvuloplasty of aortic stenosis according to claim 1, and in particular concerns an aortic valvuloplasty balloon that is covered by and elutes an antirestenosis substance (drug) to the aortic valve tissues upon contact with them during its inflation. The drug is released to the aortic valve tissues and exerts its antirestenotic action. The same technology used in the aforementioned balloon catheter (DIOR) or any other drug-covering technology (mechanical or chemical bonding of the drug to the balloon surface) is applied in manufacturing of this balloon. According to the invention, only the surfaces of the middle segment of the balloon are coated with the antirestenotic drug, i,e, the surfaces coming into direct contact to the aortic valve and the edges remain uncovered (Figure 3)].

The time of the balloon inflation in BAV cannot exceed 10-15 seconds, but multiple balloon inflations can be applied to reach a total time of balloon-valve contact of almost 1 minute. This allows the almost complete release of the loaded drug to the contacted tissue. If necessary, the quantity of the drug administered at the valve tissues by the balloon may be increased by storing higher dose of the drug at the balloon surface with appropriate technology (i.e. more and/or deeper and/or larger micropores).

A pilot trial in animals is designed to prove the concept of local drug delivery at the aortic leaflets followed by studies with experimental models of animal aortic valve stenosis and ultimately studies in humans with aortic valve stenosis.

This balloon will achieve contact and drug release at the inferior/external surfaces of the aortic valve, which have considerably larger area compared to the superior/internal surface for geometrical reasons.

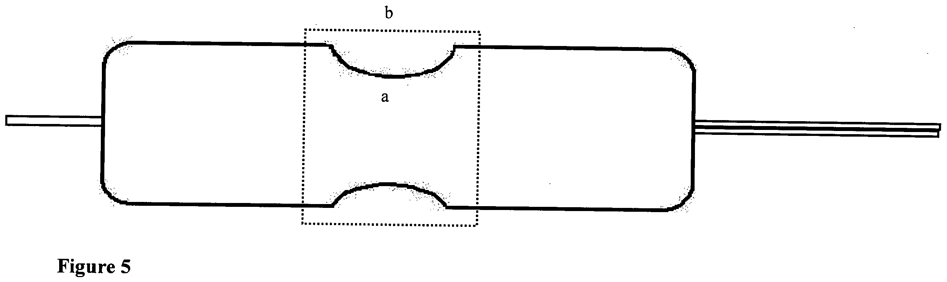

The safety of drug delivery at other endothelial sites in the vicinity (i.e. aortic root and left ventricular outflow tract-LVOT) will be assessed. In order to avoid concerns about possible side effects of paclitaxel or other drugs apposition at the aortic root/LVOT endothelium, the balloon is hour-glass shaped with the drug loaded only in the middle slimmer part (waist) of the balloon. With such a design the drug will be administered in a targeted manner at the aortic valve tissue only, and contact with other structures will be avoided. In addition, such a shape will act protectively with regards to any premature release of the drug into the blood flow. The balloon when inflated may form a circular crease at its middle part, the surface of which is covered by the drug (Figure 4). In BAV the pathological aortic valve contacts this balloon at the level of its drug-covered crease. Claim 2 describes a balloon similar to the above, the only difference being that instead of forming a crease it forms a circular concave perimeter (Figure 5).

All previously described balloons can be used for valvuloplasty of other heart valves. Phosphate binding drugs could be used for inhibition of the valve/vascular calcification component of restenosis in these studies.

Other antirestenotic and anticalcification drugs (alone or in combination) can be tested for local delivery with a coated balloon.

## Claims

1. A balloon for use in valvuloplasty of aortic stenosis comprising a catheter having two lumen running through its entire shaft and reaching to its external end that remains outside of the patient's body: wherein one lumen is used for the inflation of the balloon that is mount to the other end of the catheter, and the other lumen is used for passing a guide-wire over which the balloon is advanced from the entry site to the application site; wherein the balloon is shaped in the form of an hour-glass when inflated with its waist having a smaller diameter than of its ends, and wherein an antirestenotic drug is delivered to aortic valve tissues **characterised in that**,
the drug against restenosis coats a portion of the external surface of the balloon,
wherein only the surfaces of the middle segment of the balloon are coated with the antirestenotic drug, and
the antirestenotic drug is delivered from the external surface of the balloon during the inflation of the balloon in performing valvuloplasty.

2. The balloon of claim 1, wherein the waist has a concave shape.

## Patentansprüche

1. Ballon zur Verwendung in der Valvuloplastie einer Aortenstenose umfassend einen Katheter mit zwei Lumen, die durch dessen gesamten Schaft bis zum äußeren Ende verlaufen, das außerhalb des Patienten verbleibt, wobei das eine Lumen zur Inflation des Ballon, der am anderen Ende des Katheters angeordnet ist, dient und das andere Lumen zur Aufnahme eines Führungsdrahtes vorgesehen ist, über den der Ballon von der Einführstelle zum Zielort vorgeschoben wird, wobei der inflatierte Ballon die Form einer Sanduhr aufweist mit einer Taille, die einen geringeren Durchmesser hat als seine Enden und wobei ein antirestenotischer Wirkstoff an das Gewebe der Aortenklappe abgegeben wird, **dadurch gekennzeichnet, dass** der antirestenotische Wirkstoff einen Teil der äußeren Oberfläche des Ballons bedeckt, wobei nur die Oberfläche des mittleren Segments des Ballons mit dem antirestenotischen Wirkstoff bedeckt ist und der antirestenotische Wirkstoff während der Inflation des Ballons bei der Durchführung einer Valvuloplastie abgegeben wird.

2. Ballon nach Anspruch 1, wobei die Taille eine konkave Form hat.

## Revendications

1. Ballon destiné à être utilisé dans une valvuloplastie du rétrécissement aortique, le ballon comprenant un cathéter comportant deux lumières passant à travers tout son arbre et atteignant son extrémité externe, laquelle reste à l'extérieur du corps du patient ; une lumière étant utilisée pour le gonflage du ballon monté au niveau de l'autre extrémité du cathéter, et l'autre lumière étant utilisée pour passer un fil guide sur lequel le ballon est avancé du lieu d'entrée jusqu'au lieu d'application ; le ballon prenant la forme d'un sablier lorsqu'il est gonflé, sa constriction présentant un diamètre plus petit que celui de ses extrémités ; et un médicament antiresténotique étant administré aux tissues de la valve aortique,
**caractérisé en ce que**
le médicament contre la resténose recouvre une partie de la surface externe du ballon,
seulement les surfaces du segment central du ballon sont recouvertes avec le médicament antiresténotique, et
le médicament antiresténotique est administré à partir de la surface externe du ballon pendant le gonflage du ballon lors de la réalisation de la valvuloplastie.

2. Le ballon de la revendication 1, dans lequel la constriction présente une forme concave.
